# EUROPEAN PATENT SPECIFICATION

(11) **EP 3 681 502 B1**
(45) Date of publication and mention of the grant of the patent: **17.03.2021**
(21) Application number: 19802016.6
(22) Date of filing: 25.10.2019
(51) Int. Cl.: A61K 31/454, A61P 1/08

(54) **FORMULATIONS CONTAINING DOMPERIDONE**
ZUBEREITUNGEN ENTHALTEND DOMPERIDON
COMPOSITIONS DE DOMPÉRIDONE

(30) Priority: 25.10.2018 US 201862750480 P
(43) Date of publication of application: 22.07.2020
(73) Proprietor: CinDome Pharma, Inc., Cincinnati, OH 45227 (US)
(72) Inventor: PATEL, Piyush, Garnet Valley, Pennsylvania 19060 (US); PEARCE, Catherine, Montgomery, Ohio 45242 (US); ISAACSOHN, Jonathan, Cincinnati, Ohio 45237 (US)
(74) Representative: Høiberg P/S
(86) International application number: PCT/US2019/058037
(87) International publication number: WO 2020/086950

(56) References cited:
- CN-A- 102 125 528
- US-A1- 2005 220 825
- US-A1- 2017 298 046

## Description

### CROSS-REFERENCE TO RELATED APPLICATIONS

This application claims the benefit of U.S. Provisional Patent Application No. 62/750,480, filed October 25, 2018.

### TECHNICAL FIELD

This disclosure relates to pharmaceutical formulations containing domperidone.

### BACKGROUND

Gastroparesis is a condition where motility of the stomach does not function or does not function properly, which prevents the stomach from emptying and interferes with digestion. Treatment of gastroparesis requires medications, *e.g*., metoclopramide, erythromycin, or cisapride, to stimulate the stomach muscles. Metoclopramide poses serious side effects, such as development of movement disorders or adverse interactions with other medications; erythromycin is susceptible to loss of efficacy as patient drug tolerance increases; and cisapride has limited accessibility.

Medications to control nausea and vomiting, *e.g*., prochlorperazine, thiethylperazine, diphenhydramine, or ondansetron, may also be administered to treat gastroparesis. The symptoms of gastroparesis also may be treated surgically, such as installing jejunostomy tubes, gastric venting tubes, or feeding tubes.

Domperidone is an effective dopamine antagonist that does not readily cross the blood-brain barrier and may be used to treat gastroparesis. Pharmaceutical compositions comprising domperidone are, for instance, disclosed in CN 102125528 A. Safe and efficacious formulations of domperidone are needed.

### SUMMARY

In some embodiments, the disclosure provides pharmaceutical formulation comprising domperidone or a pharmaceutically acceptable salt thereof; a glyceryl stearate, and a medium chain triglyceride.

In other embodiments, the disclosure provides pharmaceutical formulations comprising domperidone or a pharmaceutically acceptable salt thereof; a stearoyl polyoxyl glyceride, a nonionic poly(ethylene oxide) polymer, and a medium chain triglyceride.

In further embodiments, the disclosure provides pharmaceutical formulations comprising domperidone or a pharmaceutically acceptable salt thereof; a nonionic poly(ethylene oxide) polymer, and a polyethylene glycol.

In still other embodiments, the disclosure provides methods for treating a disorder that is gastroparesis, nausea apart from gastroparesis, vomiting apart from gastroparesis, nausea associated with gastroparesis, vomiting associated with gastroparesis, gastroesophageal reflux disease, insufficient lactation, or a combination thereof in a patient, comprising administering to the patient a formulation described herein. In some aspects, the disorder is gastroparesis. In other aspects, the disorder is gastroesophageal reflux disease. In further aspects, the disorder is insufficient lactation.

Other aspects and embodiments of the invention will be readily apparent from the following detailed description of the invention.

### BRIEF DESCRIPTION OF THE DRAWINGS

The present application is further understood when read in conjunction with the appended drawings. For the purpose of illustrating the subject matter, there are shown in the drawings exemplary embodiments of the subject matter; however, the presently disclosed subject matter is not limited to the specific compositions, methods, devices, and systems disclosed. In addition, the drawings are not necessarily drawn to scale.

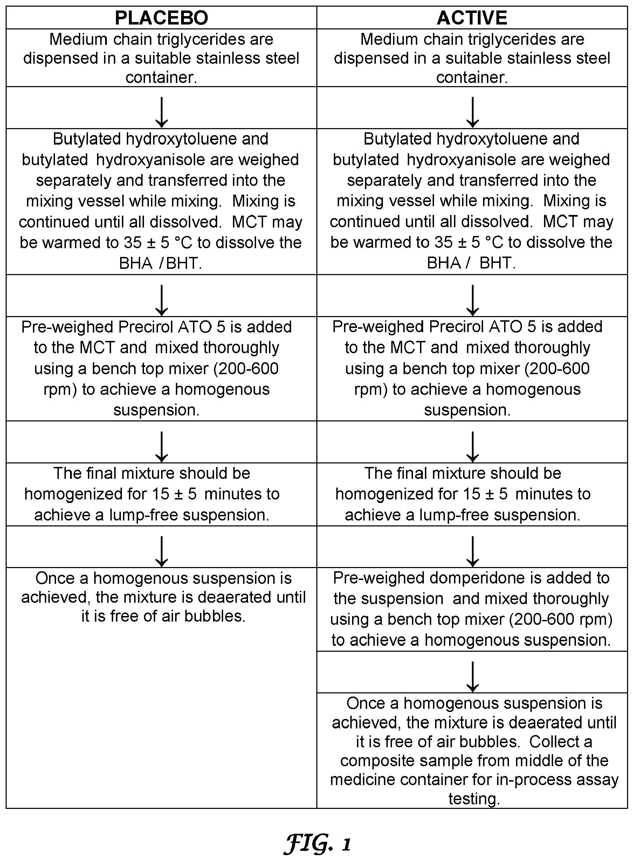

FIG. 1 is a flowchart for the fill compounding of the 5 mg and 10 mg domperidone samples.

### DETAILED DESCRIPTION OF ILLUSTRATIVE EMBODIMENTS

In the disclosure, the singular forms "a,", "an," and "the" include the plural reference, and reference to a particular numerical value includes at least that particular value, unless the context clearly indicates otherwise. Thus, for example, a reference to "a material" is a reference to at least one of such materials and equivalents thereof known to those skilled in the art, and so forth.

When a value is expressed as an approximation by use of the descriptor "about" it will be understood that the particular value forms another embodiment. In general, use of the term "about" indicates approximations that can vary depending on the desired properties sought to be obtained by the disclosed subject matter and is to be interpreted in the specific context in which it is used, based on its function. The person skilled in the art will be able to interpret this as a matter of routine. In some cases, the number of significant figures used for a particular value may be one non-limiting method of determining the extent of the word "about." In other cases, the gradations used in a series of values may be used to determine the intended range available to the term "about" for each value. Where present, all ranges are inclusive and combinable. That is, references to values stated in ranges include every value within that range.

When a list is presented, unless stated otherwise, it is to be understood that each individual element of that list and every combination of that list is to be interpreted as a separate embodiment. For example, a list of embodiments presented as "A, B, or C" is to be interpreted as including the embodiments, "A," "B," "C," "A or B," "A or C," "B or C," or "A, B, or C."

It is to be appreciated that certain features of the invention which are, for clarity, described herein in the context of separate embodiments, may also be provided in combination in a single embodiment. That is, unless obviously incompatible or excluded, each individual embodiment is deemed to be combinable with any other embodiment(s) and such a combination is considered to be another embodiment. Conversely, various features of the invention that are, for brevity, described in the context of a single embodiment, may also be provided separately or in any sub-combination. It is further noted that the claims may be drafted to exclude an optional element. As such, this statement is intended to serve as antecedent basis for use of such exclusive terminology as "solely," "only" and the like in connection with the recitation of claim elements, or use of a "negative" limitation. Finally, while an embodiment may be described as part of a series of steps or part of a more general structure, each said step may also be considered an independent embodiment in itself.

The terms "subject" and "patient" are used interchangeably and typically refer to mammals. In some embodiments, the patient or subject is a human. In other embodiments, the patient or subject is a veterinary or farm animal, a domestic animal or pet, or animal used for conducting clinical research.

"Treating" or variations thereof refers to eliminating or reducing at least one physical parameter of the disease or disorder.

"Domperidone" as referenced herein refers to 5-chloro-1-(1-[3-(2-oxo-2,3-dihydro-1H-benzo[d]imidazol-1-yl)propyl]piperidin-4-yl)-1H-benzo[d]imidazol-2(3H)-one, which has the following structure, wherein all atoms are present in their naturally-occurring amounts:

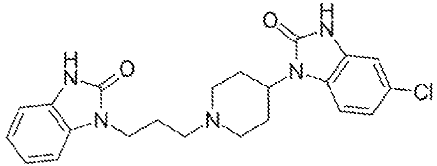

Any reference to domperidone may also include, where noted, pharmaceutically acceptable salts, esters, hydrates, solvates, prodrug forms, and derivatives of these, which are broadly defined as domperidone compounds that are modified or partially substituted, examples include but are not limited to adding a single atom, adding a reactive group, adding a functional group, forming a dimer or multimer, conjugating to another molecule such as an antibody, etc.

"Pharmaceutically acceptable" refers to properties and/or substances that are acceptable to the patient from a pharmacological/toxicological vantage, and to the manufacturing pharmaceutical chemist from a physical/chemical vantage regarding composition, formulation, stability, patient acceptance, and bioavailability.

A pharmaceutically acceptable salt includes salts with a pharmaceutically acceptable acid or base, e.g., inorganic acids, e.g., hydrochloric, sulfuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal, e.g. sodium or potassium, and alkali earth metal, e.g. calcium or magnesium, hydroxides, and organic bases, e.g., alkyl amines, arylalkyl amines and heterocyclic amines.

Pharmaceutical formulations containing domperidone described herein exhibit a variety of unexpected effects when administered to *in vivo.* In some embodiments, these pharmaceutical formulations result in a reduced Cₘₐₓ as compared to other domperidone formulations in the art. In other embodiments, these pharmaceutical formulations result in a lowering of the AUC as compared to other domperidone formulations in the art. In further embodiments, these pharmaceutical formulations result in a reduced Cₘₐₓ and a comparable AUC. In addition, as disclosed herein, the described domperidone formulations have much higher bioavailability as compared to other domperidone formulations in the art.

The pharmaceutical formulations contain about 1 to about 20% (w/w), based on the weight of the formulation, of domperidone. In some embodiments, the pharmaceutical formulations contain about 2 to about 19% (w/w), about 3 to about 18% (w/w), about 4 to about 17% (w/w), about 5 to about 16% (w/w), about 6 to about 15% (w/w), about 7 to about 15% (w/w), about 8 to about 14% (w/w), about 9 to about 13% (w/w), about 10 to about 12% (w/w), about 5 to about 15% (w/w), about 5 to about 14% (w/w), about 5 to about 13% (w/w), about 5 to about 12% (w/w), about 5 to about 11% (w/w), about 5 to about 10% (w/w), about 5 to about 9% (w/w), about 1 to about 15% (w/w), or about 1 to about 10% (w/w) of domperidone. In other embodiments, the pharmaceutical formulations contain about 5 to about 12% (w/w) of domperidone. In further embodiments, the pharmaceutical formulations contain about 1, about 2, about 3, about 4, about 5, about 6, about 7, about 8, about 9, about 10, about 11, about 12, about 13, about 14, about 15, about 16, about 17, about 18, about 19, or about 20% (w/w) of domperidone. In still other embodiments the pharmaceutical formulations contain about 10% (w/w) of domperidone.

The amount of domperidone in the pharmaceutical formulations may also be expressed by way of an amount. In some embodiments, the pharmaceutical formulations contain about 1 to about 50 mg of domperidone. In other embodiments, the pharmaceutical formulations contain about 5 to about 45 mg, about 10 to about 40 mg, about 15 to about 35 mg, about 20 to about 30 mg, about 1 to about 45 mg, about 1 to about 40 mg, about 1 to about 35 mg, about 1 to about 30 mg, about 1 to about 25 mg, about 1 to about 20 mg, about 1 to about 15 mg, about 5 to about 50 mg, about 5 to about 40 mg, about 5 to about 35 mg, about 5 to about 30 mg, about 5 to about 25 mg, about 5 to about 20 mg, about 5 to about 15 mg, about 10 to about 50 mg, about 20 to about 50 mg, about 30 to about 50 mg, or about 40 to about 50 mg of domperidone. In further embodiments, the pharmaceutical formulations contain about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, or about 50 mg of domperidone.

### Domperidone Formulations Containing a Glyceryl Stearate and Medium Chain Triglyceride

In some embodiments, the present disclosure provides pharmaceutical formulations comprising domperidone or a pharmaceutically acceptable salt thereof, a glyceryl stearate, and a medium chain triglyceride.

As one component, the pharmaceutical formulations contain a glyceryl stearate. The term "glyceryl stearate" as used herein refers to a compound having glyceryl and stearate components as shown below, where the components are bound together to form a chemically stable molecule.

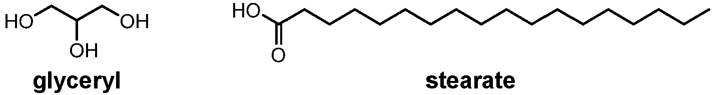

In some embodiments, the glyceryl stearate is a glyceryl palmitostearate. In other embodiments the glyceryl stearate is a glycerol distearate. In further embodiments, the glyceryl stearate is a glyceryl distearate. In yet other embodiments, the pharmaceutical formulation may contain combinations of glyceryl stearates. Thus, the pharmaceutical formulation may contain 1, 2, 3, 4, or more glyceryl stearates. In some embodiments, the pharmaceutical formulations contain glyceryl palmitostearate and glycerol distearate. In other embodiments, the pharmaceutical formulations contain glyceryl palmitostearate and glyceryl distearate. In further embodiments, the pharmaceutical formulations contain glycerol distearate and glyceryl distearate. In yet other embodiments, the pharmaceutical formulations contain glyceryl palmitostearate, glycerol distearate, and glyceryl distearate. In still other embodiments, the glyceryl stearate is Precirol® ATO 5.

The pharmaceutical formulations contain about 2 to about 20% (w/w), based on the weight of the formulation, of the glyceryl stearate. In some embodiments, the pharmaceutical formulations contains about 5 to about 15% (w/w) of the glyceryl stearate. In further embodiments, the pharmaceutical formulations contains about 6 to about 14% (w/w), about 7 to about 13% (w/w), about 8 to about 12% (w/w), about 9 to about 11% (w/w), or 5 to about 15% (w/w) of the glyceryl stearate. In other embodiments, the pharmaceutical formulations contains about 5, about 6, about 7, about 8, about 9, about 10, about 11, about 12, about 13, about 14, or about 15% (w/w) of the glyceryl stearate. In yet further embodiments, the pharmaceutical formulations contain about 9% (w/w) of the glyceryl stearate. In still other embodiments, the pharmaceutical formulations contain about 10% (w/w) of the glyceryl stearate.

The term "medium chain triglyceride" as used herein refers to triglycerides where the fatty acid moiety have an aliphatic tail of about 6 to about 12 carbon atoms. In some embodiments, the fatty acid moiety has an aliphatic tail of 6, 7, 8, 9, 10, 11, or 12 carbon atoms. In further embodiments, the fatty acid aliphatic tails are the same. In other embodiments, the fatty acid aliphatic tails are different. In still further embodiments, the fatty acid has an aliphatic tail of 6 carbon atoms, *i.e.,* the medium chain triglyceride is caproic acid. In yet other embodiments, the fatty acid has an aliphatic tail of about 8 carbon atoms, *i.e.,* the medium chain triglyceride is caprylic acid. In other embodiments, the fatty acid has an aliphatic tail of about 10 carbon atoms, *i.e.,* the medium chain triglyceride is capric acid. In further embodiments, the fatty acid has an aliphatic tail of about 12 carbon atoms, *i.e.,* the medium chain triglyceride is lauric acid.

The medium chain triglyceride is present in the pharmaceutical formulation at about 70 to about 90% (w/w), based on the weight of the formulation. In some embodiments, the pharmaceutical formulations contains about 72 to about 88% (w/w), about 74 to about 86 % (w/w), about 76 to about 84 % (w/w), about 78 to about 82% (w/w), about 70 to about 85% (w/w), about 70 to about 80% (w/w), about 75 to about 90% (w/w), about 75 to about 85% (w/w), about 75 to about 80% (w/w), about 80 to about 90% (w/w), about 80 to about 85% (w/w), or about 80 to about 90% (w/w) of the medium chain triglyceride. In further embodiments, the pharmaceutical formulations contain about 80 to about 85% (w/w) of the medium chain triglyceride. In other embodiments, the pharmaceutical formulations contain about 70, about 71, about 72, about 73, about 74, about 75, about 76, about 77, about 78, about 79, about 80, about 81, about 82, about 83, about 84, about 85, about 86, about 87, about 88, about 89, or about 90% (w/w) of the medium chain triglyceride. In yet further embodiments, the pharmaceutical formulations contain about 90% (w/w) of the medium chain triglyceride. In still other embodiments, the pharmaceutical formulations contain about 91% (w/w) of the medium chain triglyceride.

In some aspects, the pharmaceutical formulations contain about 1 to about 20% (w/w), of domperidone or a pharmaceutically acceptable salt thereof, about 2 to about 20% (w/w) of the glyceryl stearate and about 70 to about 90% (w/w) of the medium chain triglyceride. In other aspects, the pharmaceutical formulations contain about 5 to about 15% (w/w) of domperidone or a pharmaceutically acceptable salt thereof, about 5 to about 15% (w/w) of the glyceryl stearate, and about 80 to about 85% (w/w) of the medium chain triglyceride. In further aspects, the pharmaceutical formulations contain about 10% (w/w) of the glyceryl stearate and about 90% (w/w) of the medium chain triglyceride.

### Domperidone Formulation Containing a Stearoyl Polyoxyl Glyceride, Nonionic Poly(ethylene oxide) Polymer, and Medium Chain Triglyceride

The present disclosure also provides pharmaceutical formulations comprising domperidone or a pharmaceutically acceptable salt thereof, a stearoyl polyoxyl glyceride, a nonionic poly(ethylene oxide) polymer, and a medium chain triglyceride.

As a first component, the pharmaceutical formulations contain a stearoyl polyoxyl glyceride. The term "stearoyl polyoxyl glyceride" as used herein refers to a mixture of glycerol esters and polyethylene glycol. Typically, the polyethylene glycol has a mean molecular weight (Mₙ) of about 350 to about 1700. In some embodiments, the polyethylene glycol has a Mₙ of about 400 to about 1500, about 500 to about 1400, about 600 to about 1300, about 700 to about 1200, about 800 to about 1100, about 400 to about 1300, about 400 to about 1100, about 400 to about 900, about 400 to about 700, about 500 to about 1500, about 700 to about 1500, about 900 to about 1500, about 1100 to about 1500, or about 1300 to about 1500. In other embodiments the polyethylene glycol has a Mₙ of about 400, about 450, about 500, about 550, about 600, about 650, about 700, about 750, about 800, about 850, about 900, about 950, about 1000, about 1050,about 1100, about 1150, about 1200, about 1250, about 1300, about 1350, about 1400, about 1450, about 1500, about 1550, about 1600, about 1650, or about 1700. In further embodiments, the polyethylene glycol has a Mₙ of about 1450 to about 1550. In still other embodiments, the mixture of polyethylene glycol and glycerol esters is a Gelucire® product (available from Gattefosse) such as Gelucire® 44/14 (containing mono, di- and triglycerides and PEG-32 (molecular weight of about 1450 to about 1550) mono- and diesters of lauric acid (C₁₂) having a melting range of about 42.5 to about 47.5°C, or critical micelle concentration (CMC) of 72 ± 53g/mL at about 25°C), Gelucire® 50/13 (containing mono, di- and triglycerides and PEG-32 (Mₙ of about 1450 to about 1550) mono- and diesters of palmitic (C₁₆) and stearic (C₁₈) acids, a melting range of about 46 to about 51°C, hydrophile-lipophile balance (HLB) of about 13, CMC of about 100 mg/L at about 25°C), Gelucire® 43/01 (containing mono-, di- and triglyceride esters of fatty acids (C₈₋₁₈), a melting range of about 42 to about 46°C, and/or HLB of about 1), or Gelucire® 48/16 (containing PEG-32 (molecular weight of about 1450 to about 1550) esters of fatty acids, a melting range of about 46 to about 50°C, HLB of about 16, and/or CMC of 153 ± 31 mg/L at about 25°C). In some embodiments, a stearoyl polyoxyl glyceride contains monoesters, diesters, and triesters of glycerol. In other embodiments, a stearoyl polyoxyl glyceride contains monoesters and diesters of polyethylene glycols. In further embodiments, the stearoyl polyoxyl glyceride contains (i) monoesters, diesters, and/or triesters of glycerol and (ii) monoesters and/or diesters of polyethylene glycols. In yet other embodiments, the stearoyl polyoxyl glyceride is a stearoyl polyoxyl-32 glyceride (containing 32 repeating oxyethylene units). In still further embodiments, the stearoyl polyoxyl glyceride is Gelucire® 50/13.

The pharmaceutical formulations contain about 3 to about 15% (w/w), based on the weight of the formulation, of the stearoyl polyoxyl glyceride. In some embodiments, the pharmaceutical formulations contain about 4 to about 14% (w/w), about 5 to about 13% (w/w), about 6 to about 12% (w/w), about 7 to about 11% (w/w), about 8 to about 10% (w/w), about 5 to about 12% (w/w), about 5 to about 10% (w/w), or about 6 to about 8% (w/w) of the stearoyl polyoxyl glyceride. In other embodiments, the pharmaceutical formulations contain about 3, about 4, about 5, about 6, about 7, about 8, about 9, about 10, about 11, about 12, about 13, about 14, or about 15% (w/w) of the stearoyl polyoxyl glyceride. In further embodiments, the pharmaceutical formulations contain about 7% (w/w) of the stearoyl polyoxyl glyceride.

The pharmaceutical formulation also contains a nonionic poly(ethylene oxide) polymer. The term "nonionic poly(ethylene oxide)" as used herein refers to a polymer having the following structure that is a liquid at room temperature. 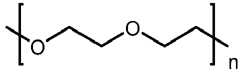 In some embodiments, n is about 2,000 to about 100,000. In other embodiments, n is about 2,000 to about 90,000; about 2,000 to about 80,000; about 2,000 to about 60,000; about 2,000 to about 40,000; about 2,000 to about 20,000; about 2,000 to about 10,000; about 2,000 to about 8,000; about 2,000 to about 6,000; about 2,000 to about 4,000; about 4,000 to about 100,000; about 8,000 to about 100,000; about 10,000 to about 100,000; about 20,000 to about 100,000; about 40,000 to about 100,000; about 60,000 to about 100,000; about 80,000 to about 100,000; about 4,000 to about 80,000; about 6,000 to about 60,000; about 8,000 to about 40,000; about 10,000 to about 20,000. In further embodiments, n is about 2,000; 3,000; 4,000; 5,000; 6,000; 7,000; 8,000; 9,000; 10,000; 15,000; 20,000; 25,000; 30,000; 35,000; 40,000; 45,000; 50,000; 55,000; 60,000; 65,000; 70,000; 75,000; 80,000; 85,000; 90,000, 95,000; or 100,000.

In some embodiments, the nonionic poly(ethylene oxide) polymer has a molecular weight (M_{w}) of about 400,000 to about 8,000,000. In other embodiments, the nonionic poly(ethylene oxide) has a M_{w} of about 500,000 to about 8,000,000, about 600,000 to about 8,000,000, about 700,000 to about 8,000,000, about 800,000 to about 8,000,000, about 900,000 to about 8,000,000, about 1,000,000 to about 8,000,000, about 2,000,000 to about 8,000,000, about 3,000,000 to about 8,000,000, about 4,000,000 to about 8,000,000, about 5,000,000 to about 8,000,000, about 6,000,000 to about 8,000,000. In further embodiments, the nonionic poly(ethylene oxide) polymer has a M_{w} of about 500,000, about 600,000, about 700,000, about 800,000, about 900,000, about 1,000,000, about 2,000,000, about 3,000,000, about 4,000,000, about 5,000,000, about 6,000,000, about 7,000,000, or about 8,000,000. In yet other embodiments, the nonionic poly(ethylene oxide) has a M_{w} of about 7,000,000. In yet other embodiments, the nonionic poly(ethylene oxide) is polyethylene oxide 303. In still further embodiments, the nonionic poly(ethylene oxide) is POLYOX™ WSR 303.

The nonionic poly(ethylene oxide) polymer is present in the pharmaceutical formulations at about 5 to about 40% (w/w), based on the weight of the formulation. In some embodiments, the pharmaceutical formulations contain about 10 to about 35% (w/w), about 15 to about 30% (w/w), about 20 to about 25% (w/w), about 5 to about 35% (w/w), about 5 to about 30% (w/w), about 5 to about 25% (w/w), about 5 to about 20% (w/w), about 5 to about 15% (w/w), about 5 to about 10% (w/w), about 10 to about 40% (w/w), about 10 to about 30% (w/w), about 10 to about 20% (w/w), about 10 to about 25% (w/w), about 5 to about 35 % (w/w), about 5 to about 30% (w/w), about 5 to about 25% (w/w), or about 5 to about 20% (w/w) of the nonionic poly(ethylene oxide) polymer. In other embodiments, the pharmaceutical formulations contain about 12 to about 25% (w/w) of the nonionic poly(ethylene oxide) polymer. In other embodiments, the pharmaceutical formulations contain about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40% (w/w) of the nonionic poly(ethylene oxide) polymer. In further embodiments, the pharmaceutical formulations contain about 19% (w/w) of the nonionic poly(ethylene oxide) polymer.

The pharmaceutical formulations further contain a medium chain triglyceride as defined above. In some embodiments, the fatty acid moiety has an aliphatic tail of about 6, 7, 8, 9, 10, 11, or 12 carbon atoms. In further embodiments, the medium chain triglyceride is caproic acid. In other embodiments, the medium chain triglyceride is caprylic acid. In yet further embodiments, the medium chain triglyceride is capric acid. In still other embodiments, the medium chain triglyceride is lauric acid.

The medium chain triglyceride is present in the pharmaceutical formulation at about 40 to about 80% (w/w), based on the weight of the formulation. In some embodiments, the pharmaceutical formulations contains about 45 to about 75% (w/w), about 50 to about 70% (w/w), about 55 to about 65% (w/w), about 50 to about 80% (w/w), about 60 to about 80% (w/w), about 70 to about 80% (w/w), about 40 to about 70% (w/w), about 40 to about 60% (w/w), or about 50 to about 70% (w/w) of the medium chain triglyceride. In further embodiments, the pharmaceutical formulations contain about 50 to about 68% (w/w) of the medium chain triglyceride. In other embodiments, the pharmaceutical formulations contain about 40, about 41, about 42, about 43, about 44, about 45, about 46, about 47, about 48, about 49, about 50, about 51, about 52, about 53, about 54, about 55, about 56, about 57, about 58, about 59, about 60, about 61, about 62, about 63, about 64, about 65, about 66, about 67, about 68, about 69, about 70, about 71, about 72, about 73, about 74, about 75, about 76, about 77, about 78, about 79, or about 80% (w/w) of the medium chain triglyceride. In still further embodiments, the pharmaceutical formulations contain about 64% (w/w) of the medium chain triglyceride.

In some aspects, the pharmaceutical formulations contain about 1 to about 20% (w/w), based on the weight of the formulation, of domperidone or a pharmaceutically acceptable salt thereof, about 3 to about 15% (w/w) stearoyl polyoxyl glyceride, about 5 to about 40% (w/w) of the nonionic poly(ethylene oxide) polymer, and about 40 to about 80% (w/w) of the medium chain triglyceride. In other aspects, the pharmaceutical formulations contain about 5 to about 10% (w/w), based on the weight of the formulation, of domperidone or a pharmaceutically acceptable salt thereof, about 5 to about 10% (w/w) stearoyl polyoxyl glyceride, about 12 to about 25% (w/w) of the nonionic poly(ethylene oxide) polymer, and about 50 to about 68% (w/w) of the medium chain triglyceride.

### Domperidone Formulation Containing a Nonionic Poly(ethylene oxide) Polymer and Polyethylene Glycol

In further embodiments, the present disclosure provides pharmaceutical formulations comprising domperidone or a pharmaceutically acceptable salt thereof, a nonionic poly(ethylene oxide) polymer, and a polyethylene glycol.

As one component, the pharmaceutical formulations contain a nonionic poly(ethylene oxide) polymer as defined above. In some embodiments, the nonionic poly(ethylene oxide) is polyethylene oxide 303. In further embodiments, the nonionic poly(ethylene oxide) is POLYOX™ WSR 303.

The nonionic poly(ethylene oxide) polymer is present in the pharmaceutical formulations at about 5 to about 30% (w/w), based on the weight of the formulation. In some embodiments, the pharmaceutical formulations contain about 10 to about 30% (w/w), about 15 to about 30% (w/w), about 20 to about 25% (w/w), about 5 to about 30% (w/w), about 5 to about 25% (w/w), about 5 to about 20% (w/w), about 5 to about 15% (w/w), about 5 to about 10% (w/w), about 10 to about 20% (w/w), about 10 to about 25% (w/w), or about 5 to about 20% (w/w) of the nonionic poly(ethylene oxide) polymer. In other embodiments, the pharmaceutical formulations contain about 10 to about 20% (w/w) of the nonionic poly(ethylene oxide) polymer. In further embodiments, the pharmaceutical formulations contain about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30% (w/w) of the nonionic poly(ethylene oxide) polymer. In still other embodiments, the pharmaceutical formulations contain about 15% (w/w) of the nonionic poly(ethylene oxide) polymer.

The pharmaceutical formulations also contain polyethylene glycol. The term "polyethylene glycol" as used herein refers to chemical compound having the following structure that is a liquid at room temperature, wherein m is about 7 to about 20. 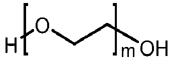 In some embodiments, m is about 7 to about 15, about 7 to about 10, about 8 to about 15, about 8 to about 10, about 9 to about 15, or about 9 to about 13. In other embodiments, m is about m is about 8 to 9. In further embodiments, m is about 7, 8, 9, 10, 11, 12, 13, 14, 15, 16, 17, 18, 19, or about 20. In yet further embodiments, m is about 8. In still other embodiments, m is about 9. The polyethylene glycol has also a molecular weight (M_{w}) of about 300 to about 1,000. In some embodiments, the polyethylene glycol has a M_{w} of about 400 to about 900, about 500 to about 800, about 600 to about 700, about 300 to about 900, about 300 to about 800, about 300 to about 700, about 300 to about 600, about 300 to about 500, about 400 to about 1,000, about 500 to about 1,000, about 600 to about 1,000, about 700 to about 1,000, about 800 to about 1,000, or about 900 to about 1,000. In further embodiments, the polyethylene glycol has a M_{w} of about 400, about 450, about 500, about 550, 6 about 00, about 650, about 700, about 750, about 800, about 850, about 900, about 950, or about 1,000. In yet other embodiments, the polyethylene glycol has a M_{w} of about 400. In yet other embodiments, the polyethylene glycol is polyethylene glycol 400.

The pharmaceutical formulations contain about 70 to about 90% (w/w), based on the weight of the formulation, of the polyethylene glycol. In some embodiments, the pharmaceutical formulations contain about 70 to about 85% (w/w), about 70 to about 80% (w/w), about 70 to about 75% (w/w), about 75 to about 90% (w/w), about 80 to about 90% (w/w), or about 85 to about 90% (w/w) of the polyethylene glycol. In other embodiments, the pharmaceutical formulations contain about 70, about 71, about 72, about 73, about 74, about 75, about 76, about 77, about 78, about 79, about 80, about 81, about 82, about 83, about 84, about 85, about 86, about 87, about 88, about 89, or about 90% (w/w). In further embodiments, the pharmaceutical formulations contain about 75% (w/w) of the polyethylene glycol.

In some aspects, the pharmaceutical formulations contain about 1 to about 20% (w/w), based on the weight of the formulation, of domperidone or a pharmaceutically acceptable salt thereof, about 5 to about 30% (w/w) of the nonionic poly(ethylene oxide) polymer and about 70 to about 90% (w/w) of the polyethylene glycol. In other aspects, the pharmaceutical formulations contain about 5 to about 10% (w/w), based on the weight of the formulation, of domperidone or a pharmaceutically acceptable salt thereof, about 10 to about 20% (w/w) of the nonionic poly(ethylene oxide) polymer and about 70 to about 80% (w/w) of the polyethylene glycol.

The pharmaceutical formulations described herein may also contain one or more antioxidants. In some embodiments, the pharmaceutical formulations contain one antioxidant. In other embodiments, the pharmaceutical formulations contain two antioxidants. In further embodiments, the pharmaceutical formulations contain three antioxidants. The antioxidant may be selected by those skilled in the art. In some embodiments, the antioxidant is ascorbic acid, ascorbyl palmitate, butylated hydroxyanisole (BHA), butylated hydroxytoluene (BHT), propyl gallate, potassium metabisulfite, sodium metabisulfite, sodium thiosulfate, or vitamin E. In other embodiments, the antioxidant is BHA. In further embodiments, the antioxidant is BHT. In still other embodiments, the antioxidant is BHA and BHT.

The total amount of antioxidant in the pharmaceutical formulation is about 0.01 to about 0.5% (w/w), of the total weight of the formulation. In some embodiments, amount of one or more antioxidant in the pharmaceutical formulation is about 0.01 to about 0.4, about 0.01 to about 0.3, about 0.01 to about 0.2, about 0.01 to about 0.1, about 0.01 to about 0.05, about 0.05 to about 0.4, about 0.05 to about 0.3, about 0.05 to about 0.2, about 0.05 to about 0.1% (w/w). In other embodiments, the pharmaceutical formulations contain about 0.01, about 0.02, about 0.03, about 0.04, about 0.05, about 0.06, about 0.07, about 0.08, about 0.09, about 0.10, about 0.11, about 0.12, about 0.013, about 0.014, about 0.015, about 0.016, about 0.017, about 0.018, about 0.019, about 0.20, about 0.21, about 0.22, about 0.23, about 0.24, about 0.25, about 0.26, about 0.27, about 0.28, about 0.29, about 0.30, about 0.31, about 0.32, about 0.33, about 0.34, about 0.35, about 0.36, about 0.37, about 0.38, about 0.39, about 0.40, about 0.41, about 0.42, about 0.43, about 0.44, about 0.45, about 0.46, about 0.47, about 0.48, about 0.49, or about 0.50% (w/w). In further embodiments, the pharmaceutical formulations contain about 0.1% (w/w) of the antioxidant. In still other embodiments, the pharmaceutical formulations contain about 0.05% (w/w) of the antioxidant. In yet further embodiments, the pharmaceutical formulations contain about 0.15% (w/w) of the antioxidant. In other embodiments, the pharmaceutical formulations contain about 0.1% (w/w) BHA. In further embodiments, the pharmaceutical formulations contain about 0.05% (w/w) of BHT. In yet other embodiments, the pharmaceutical formulations contain about 0.1% (w/w) BHA and 0.05% (w/w) of BHT.

The domperidone formulations described herein are useful in a variety of treatment methods including, without limitation, methods for treating a disorder that is gastroparesis, nausea apart from gastroparesis, vomiting apart from gastroparesis, nausea associated with gastroparesis, vomiting associated with gastroparesis, gastroesophageal reflux disease, insufficient lactation, nausea and/or vomiting associated with chemotherapy, or a combination thereof. The methods include administering to the patient a pharmaceutical formulation described herein. In some embodiments, the methods are useful for treating gastroparesis. In other embodiments, the methods are useful for treating nausea apart from gastroparesis. In further embodiments, the methods are useful for treating vomiting apart from gastroparesis. In yet other embodiments, the methods are useful for treating nausea associated with gastroparesis. In still further embodiments, the methods are useful for treating vomiting associated with gastroparesis. In other embodiments the methods are useful for treating gastroesophageal reflux disease. In further embodiments, the methods are useful for treating insufficient lactation. In still other embodiments, the methods are useful for treating nausea and/or vomiting associated with chemotherapy.

The pharmaceutical formulations may be administered by any acceptable route. In some embodiments, the pharmaceutical formulations the administration is oral, transdermal, parenteral, or a combination thereof. In further embodiments, administration is oral.

The pharmaceutical formulations may be formulated for administration in solid or liquid forms. In some embodiments, the pharmaceutical formulations are formulated in the form of a tablet, caplet, capsule, powder, softgel, suspension or liquid, or a combination thereof. In other embodiments, the pharmaceutical formulations are formulated in the form of a tablet. In further embodiments, the pharmaceutical formulations are formulated in the form of a caplet. In yet other embodiments, the pharmaceutical formulations are formulated in the form of a capsule. In still further embodiments, the pharmaceutical formulations are formulated in the form of a powder. In other embodiments, the pharmaceutical formulations are formulated in the form of a softgel. In further embodiments, the pharmaceutical formulations are formulated in the form of suspension. In yet other embodiments, the pharmaceutical formulations are formulated in the form of a liquid.

The following Examples are provided to illustrate some of the concepts described within this disclosure. While each Example is considered to provide specific individual embodiments of formulations, methods of preparation and use, none of the Examples should be considered to limit the more general embodiments described herein.

In the following examples, efforts have been made to ensure accuracy with respect to numbers used (e.g. amounts, temperature, etc.) but some experimental error and deviation should be accounted for. Unless indicated otherwise, temperature is in degrees C; Cₘₐₓ = maximum plasma concentration; tmax = time of maximum plasma concentration; MRTₗₐₛₜ = mean residence time, calculated to the last observable time point; AUCₗₐₛₜ = area under the curve, calculated to the last observable time point.

### EXAMPLES

### Example 1: Formulation Preparation

Five formulations, *i.e.,* A-E, containing domperidone are prepared by combining the components identified in Table 1.

| **Table 1** | | | | | |
|---|---|---|---|---|---|
| **Ingredient** | **Formulation (%)** | | | | |
| | **A** | **B** | **c** | **D Tablet** | **E** |
| Domperidone | 10.0 | 9.6 | 10.0 | 2.5 | 4.6 |
| Precirol ATO 5 | 9.75 | 0 | 0 | 0 | 0 |
| Gelucire 50/13 | 0 | 7.2 | 0 | 0 | 0 |
| Cremophor RH40 | 0 | 0 | 0 | 0 | 13.9 |
| PEO 303 | 0 | 19.4 | 15.0 | 20.0 | 0 |
| PEG 400 | 0 | 0 | 75.0 | 0 | 6.9 |
| Avicel PH 102 | 0 | 0 | 0 | 77.5 | 0 |
| Compritol ATO 888 | 0 | 0 | 0 | 0 | 10.0 |
| Oleic acid | 0 | 0 | 0 | 0 | 64.6 |
| MCT | 80.25 | 63.8 | 0 | 0 | 0 |
| Fill/Tablet Weight (mg) | 100.0 | 104.0 | 100.0 | 400.0 | 217.0 |

Formulations A-C and E are formulated as liquid or semi solid and filled into capsules and formulation D is compressed to form a tablet using a single station carver press.

### Example 2

Domperidone pharmaceutical formulations are prepared and encapsulated on a GMP encapsulation machine using the amounts noted in Tables 2-4. Specifically, the compounding activities are conducted under a nitrogen blanket and yellow light. The batches are then encapsulated using the 2C Oval die and 0.040" hole single bottom shot wedge at a temperature of about 38.7 to 51.7°C. Capsules are hand polished with a medium chain triglyceride/lecithin mixture (97% MCT/3%lecithin).

| **Table 2: Formulation for Domperidone Placebo Softgels Fill** | | |
|---|---|---|
| **Ingredient** | **Theoretical Quantity/capsule (mg)** | **Theoretical Quantity (g)** |
| Medium Chain Triglycerides | 90.1 | 4505.0 |
| Glyceryl Distearate | 9.75 | 487.5 |
| Butylated Hydroxyanisole, NF | 0.1 | 5.0 |
| Butylated Hydroxytoluene, NF | 0.05 | 2.5 |
| Total | 100.0 | 50000 |

| **Table 3: Formulation for Domperidone 5 mg Softgels Fill** | | |
|---|---|---|
| **Ingredient** | **Theoretical Quantity/capsule (mg)** | **Theoretical Quantity (g)** |
| Domperidone | 5.0 | 100.00 |
| Medium Chain Triglycerides | 85.1 | 1702.00 |
| Glyceryl Distearate | 9.75 | 195.00 |
| Butylated hydroxyanisole, NF | 0.1 | 2.00 |
| Butylated hydroxytoluene, NF | 0.05 | 1.00 |
| Total | 100.0 | 2000.00 |

| **Table 4: Formulation for Domperidone 10 mg Softgels Fill** | | |
|---|---|---|
| **Ingredient** | **Theoretical Quantity/capsule (mg)** | **Theoretical Quantity (g)** |
| Domperidone | 10.0 | 200.00 |
| Medium Chain Triglycerides | 80.1 | 1602.00 |
| | 9.75 | 195.00 |
| Butylated Hydroxyanisole, NF | 0.1 | 2.00 |
| Butylated Hydroxytoluene NF | 0.05 | 1.00 |
| Total | 100.0 | 200000 |

It is to be understood that while the invention has been described in conjunction with the preferred specific embodiments thereof, that the foregoing description and the examples that follow are intended to illustrate and not limit the scope of the invention.

## Claims

1. A pharmaceutical formulation comprising:
(i) domperidone or a pharmaceutically acceptable salt thereof, a glyceryl stearate, and a medium chain triglyceride; or
(ii) domperidone or a pharmaceutically acceptable salt thereof, a stearoyl polyoxyl glyceride, a nonionic poly(ethylene oxide) polymer, and a medium chain triglyceride; or
(iii) domperidone or a pharmaceutically acceptable salt thereof, a nonionic poly(ethylene oxide) polymer, and a polyethylene glycol.

2. The pharmaceutical formulation of claim 1, comprising domperidone or a pharmaceutically acceptable salt thereof, a glyceryl stearate, and a medium chain triglyceride.

3. The pharmaceutical formulation of claim 1 or 2, wherein the glyceryl stearate is a glyceryl palmitostearate, a glycerol distearate, a glyceryl distearate, or a combination thereof.

4. The pharmaceutical formulation of any one of the preceding claims, comprising about 2 to about 20% (w/w), based on the weight of the formulation, of the glyceryl stearate, preferably about 5 to about 15% (w/w), or more preferably about 10% (w/w).

5. The pharmaceutical formulation of any one of the preceding claims, comprising about 70 to about 90% (w/w), based on the weight of the formulation, of the medium chain triglyceride, or preferably about 80 to about 85% (w/w).

6. The pharmaceutical formulation of any one of the preceding claims, wherein the glyceryl stearate is Precirol® ATO 5.

7. The pharmaceutical formulation of claim 1, comprising domperidone or a pharmaceutically acceptable salt thereof, a stearoyl polyoxyl glyceride, a nonionic poly(ethylene oxide) polymer, and a medium chain triglyceride.

8. The pharmaceutical formulation of claim 1, comprising domperidone or a pharmaceutically acceptable salt thereof, a nonionic poly(ethylene oxide) polymer, and a polyethylene glycol.

9. The pharmaceutical formulation of any one of the preceding claims, further comprising an antioxidant.

10. The pharmaceutical formulation of claim 9, wherein the antioxidant is ascorbic acid, ascorbyl palmitate, butylated hydroxyanisole, butylated hydroxytoluene, propyl gallate, potassium metabisulfite, sodium metabisulfite, sodium thiosulfate, or vitamin E, or preferably butylated hydroxyanisole or butylated hydroxytoluene.

11. The pharmaceutical formulation of any one of the preceding claims, comprising about 1 to about 20% (w/w), based on the weight of the formulation, of domperidone, preferably about 5 to about 12% (w/w), or more preferably about 10% (w/w).

12. The pharmaceutical formulation of any one of the preceding claims, comprising about 1 to about 50 mg of domperidone.

13. A formulation of any one of claims 1 to 12 for use in treating a disorder that is gastroparesis, nausea apart from gastroparesis, vomiting apart from gastroparesis, nausea associated with gastroparesis, vomiting associated with gastroparesis, gastroesophageal reflux disease, insufficient lactation, or a combination thereof in a subject in need thereof.

14. The formulation for use of claim 13, wherein the formulation is in the form of a tablet, capsule, softgel, suspension, liquid, or combination thereof.

15. The formulation for use of claim 13 or 14, wherein the disorder is gastroparesis.

16. The formulation for use of claims 13 or 14, wherein the disorder is gastroesophageal reflux disease.

17. The formulation for use of claims 13 or 14, wherein the disorder is insufficient lactation.

## Patentansprüche

1. Pharmazeutische Zubereitung, umfassend:
(i) Domperidon oder ein pharmazeutisch akzeptables Salz davon, ein Glycerylstearat und ein mittelkettiges Triglycerid; oder
(ii) Domperidon oder ein pharmazeutisch akzeptables Salz davon, ein Stearoylpolyoxylglycerid, ein nichtionisches Poly(ethylenoxid)polymer und ein mittelkettiges Triglycerid; oder
(iii) Domperidon oder ein pharmazeutisch akzeptables Salz davon, ein nichtionisches Poly(ethylenoxid)polymer und ein Polyethylenglycol.

2. Pharmazeutische Zubereitung nach Anspruch 1, umfassend Domperidon oder ein pharmazeutisch akzeptables Salz davon, ein Glycerylstearat und ein mittelkettiges Triglycerid.

3. Pharmazeutische Zubereitung nach Anspruch 1 oder 2, wobei das Glycerylstearat ein Glycerylpalmitostearat, ein Glyceroldistearat, ein Glyceryldistearat oder eine Kombination davon ist.

4. Pharmazeutische Zubereitung nach einem der vorstehenden Ansprüche, umfassend etwa 2 bis etwa 20 Gew.-% Glycerylstearat, basierend auf dem Gewicht der Zubereitung, bevorzugt etwa 5 bis etwa 15 Gew.-% oder besonders bevorzugt etwa 10 Gew.-%.

5. Pharmazeutische Zubereitung nach einem der vorstehenden Ansprüche, umfassend etwa 70 bis etwa 90 Gew.-% mittelkettiges Triglycerid, basierend auf dem Gewicht der Zubereitung, oder bevorzugt etwa 80 bis etwa 85 Gew.-%.

6. Pharmazeutische Zubereitung nach einem der vorstehenden Ansprüche, wobei das Glycerylstearat Precirol® ATO 5 ist.

7. Pharmazeutische Zubereitung nach Anspruch 1, umfassend Domperidon oder ein pharmazeutisch akzeptables Salz davon, ein Stearoylpolyoxylglycerid, ein nichtionisches Poly(ethylenoxid)polymer und ein mittelkettiges Triglycerid.

8. Pharmazeutische Zubereitung nach Anspruch 1, umfassend Domperidon oder ein pharmazeutisch akzeptables Salz davon, ein nichtionisches Poly(ethylenoxid)polymer und ein Polyethylenglycol.

9. Pharmazeutische Zubereitung nach einem der vorstehenden Ansprüche, ferner ein Antioxidans umfassend.

10. Pharmazeutische Zubereitung nach Anspruch 9, wobei das Antioxidans Ascorbinsäure, Ascorbylpalmitat, butyliertes Hydroxyanisol, butyliertes Hydroxytoluol, Propylgallat, Kaliummetabisulfit, Natriummetabisulfit, Natriumthiosulfat oder Vitamin E, oder bevorzugt butyliertes Hydroxyanisol oder butyliertes Hydroxytoluol ist.

11. Pharmazeutische Zubereitung nach einem der vorstehenden Ansprüche, umfassend etwa 1 bis etwa 20 Gew.-% Domperidon, basierend auf dem Gewicht der Zubereitung, bevorzugt etwa 5 bis etwa 12 Gew.-% oder besonders bevorzugt etwa 10 Gew.-%.

12. Pharmazeutische Zubereitung nach einem der vorstehenden Ansprüche, umfassend etwa 1 bis etwa 50 mg Domperidon.

13. Zubereitung nach einem der Ansprüche 1 bis 12 zur Verwendung bei der Behandlung einer Störung, die Gastroparese, Übelkeit abgesehen von Gastroparese, Erbrechen abgesehen von Gastroparese, Übelkeit in Verbindung mit Gastroparese, Erbrechen in Verbindung mit Gastroparese, gastroösophageale Refluxkrankheit, unzureichende Milchbildung, oder eine Kombination davon ist, bei einem behandlungsbedürftigen Individuum.

14. Zubereitung zur Verwendung nach Anspruch 13, wobei die Zubereitung in der Form einer Tablette, Kapsel, eines Softgels, einer Suspension, Flüssigkeit, oder einer Kombination davon vorliegt.

15. Zubereitung zur Verwendung nach Anspruch 13 oder 14, wobei die Störung Gastroparese ist.

16. Zubereitung zur Verwendung nach Anspruch 13 oder 14, wobei die Störung gastroösophageale Refluxkrankheit ist.

17. Zubereitung zur Verwendung nach Anspruch 13 oder 14, wobei die Störung unzureichende Milchbildung ist.

## Revendications

1. Formulation pharmaceutique comprenant :
(i) de la dompéridone ou un sel pharmaceutiquement acceptable de celle-ci, un stéarate de glycéryle, et un triglycéride à chaîne moyenne ; ou
(ii) de la dompéridone ou un sel pharmaceutiquement acceptable de celle-ci, un glycéride de stéaroyl polyoxyl, un polymère de poly(oxyde d'éthylène) non ionique, et un triglycéride à chaîne moyenne ; ou
(iii) de la dompéridone ou un sel pharmaceutiquement acceptable de celle-ci, un polymère de poly(oxyde d'éthylène) non ionique, et un polyéthylène glycol.

2. Formulation pharmaceutique selon la revendication 1, comprenant de la dompéridone ou un sel pharmaceutiquement acceptable de celle-ci, un stéarate de glycéryle, et un triglycéride à chaîne moyenne.

3. Formulation pharmaceutique selon la revendication 1 ou 2, dans laquelle le stéarate de glycéryle est un palmitostéarate de glycéryle, un distéarate de glycérol, un distéarate de glycéryle, ou une combinaison de ceux-ci.

4. Formulation pharmaceutique selon l'une quelconque des revendications précédentes, comprenant d'environ 2 à environ 20 % (p/p), sur la base du poids de la formulation, de stéarate de glycéryle, de préférence d'environ 5 à environ 15 % (p/p), ou de manière davantage préférée environ 10 % (p/p).

5. Formulation pharmaceutique selon l'une quelconque des revendications précédentes, comprenant d'environ 70 à environ 90 % (p/p), sur la base du poids de la formulation, du triglycéride à chaîne moyenne, de préférence d'environ 80 à environ 85 % (p/p).

6. Formulation pharmaceutique selon l'une quelconque des revendications précédentes, dans laquelle le stéarate de glycéryle est Precirol® ATO 5.

7. Formulation pharmaceutique selon la revendication 1, comprenant de la dompéridone ou un sel pharmaceutiquement acceptable de celle-ci, un glycéride de stéaroyl polyoxyl, un polymère de poly(oxyde d'éthylène) non ionique, et un triglycéride à chaîne moyenne.

8. Formulation pharmaceutique selon la revendication 1, comprenant de la dompéridone ou un sel pharmaceutiquement acceptable de celle-ci, un polymère de poly(oxyde d'éthylène) non ionique, et un polyéthylène glycol.

9. Formulation pharmaceutique selon l'une quelconque des revendications précédentes, comprenant en outre un antioxydant.

10. Formulation pharmaceutique selon la revendication 9, dans laquelle l'antioxydant est l'acide ascorbique, le palmitate d'ascorbyle, l'hydroxyanisole butylé, le hydroxytoluène butylé, le gallate de propyle, le métabisulfite de potassium, le métabisulfite de sodium, le thiosulfate de sodium, ou la vitamine E, ou de préférence l'hydroxyanisole butylé ou l'hydroxytoluène butylé.

11. Formulation pharmaceutique selon l'une quelconque des revendications précédentes, comprenant d'environ 1 à environ 20 % (p/p), sur la base du poids de la formulation, de dompéridone, de préférence d'environ 5 à environ 12 % (p/p), ou de manière davantage préférée environ 10 % (p/p).

12. Formulation pharmaceutique selon l'une quelconque des revendications précédentes, comprenant d'environ 1 à environ 50 mg de dompéridone.

13. Formulation selon l'une quelconque des revendications 1 à 12 pour son utilisation dans le traitement d'un trouble qui est une gastroparésie, des nausées sans lien avec une gastroparésie, des vomissements sans lien avec une gastroparésie, des nausées associées à une gastroparésie, des vomissements associés à une gastroparésie, une maladie du reflux gastro-œsophagien, une lactation insuffisante, ou une combinaison de ceux-ci chez un sujet le nécessitant.

14. Formulation pour son utilisation selon la revendication 13, dans laquelle la formulation se trouve sous la forme d'un comprimé, d'une capsule, d'une capsule à enveloppe molle, d'une suspension, d'un liquide, ou d'une combinaison de ceux-ci.

15. Formulation pour son utilisation selon la revendication 13 ou 14, dans laquelle le trouble est une gastroparésie.

16. Formulation pour son utilisation selon la revendication 13 ou 14, dans laquelle le trouble est une maladie du reflux gastro-œsophagien.

17. Formulation pour son utilisation selon la revendication 13 ou 14, dans laquelle le trouble est une lactation insuffisante.
